# EUROPEAN PATENT SPECIFICATION

(11) **EP 3 398 587 B1**
(45) Date of publication and mention of the grant of the patent: **02.08.2023**
(21) Application number: 16881720.3
(22) Date of filing: 26.12.2016
(51) Int. Cl.: A61K 9/20, A61K 9/50, A61K 31/192, A61K 47/32, A61K 47/34, A61K 47/38, A61P 29/00

(54) **COMPACTED PHARMACEUTICAL PREPARATION**
KOMPAKTIERTE PHARMAZEUTISCHES PRÄPARAT
PRÉPARATION PHARMACEUTIQUE OBTENUE PAR MOULAGE PAR COMPRESSION

(30) Priority: 28.12.2015 JP 2015255826
(43) Date of publication of application: 07.11.2018
(73) Proprietor: SSP Co., Ltd., Japan, Shinjuku-ku Tokyo 1631488 (JP)
(72) Inventor: ONUKI, Yoichi, Narita-shi Chiba 286-8511 (JP); OSAWA, Ryoichi, Tokyo 163-1488 (JP)
(74) Representative: Blodig, Wolfgang
(86) International application number: PCT/JP2016/088659
(87) International publication number: WO 2017/115745

(56) References cited:
- EP-A1- 1 491 184
- EP-A1- 2 719 377
- WO-A1-2014/189034
- CN-A- 103 860 464
- CN-B- 103 860 464
- JP-A- H0 253 721
- JP-A- H0 372 417
- JP-A- H0 383 922
- JP-A- H05 500 674
- JP-A- S59 227 817
- JP-A- 2007 055 924
- JP-A- 2008 231 029
- JP-A- 2009 191 034
- US-A- 5 540 945
- US-A1- 2004 265 372
- US-A1- 2008 113 021
- US-A1- 2008 113 021
- US-A1- 2010 173 921
- AKIRA ISOGAI et al.: Advanced Technologies of Cellulose Utilization, 2008, pages 160-165, ISBN: 978-4-7813-0010-8
- RATTES, ALYSSON L. R. et al.: "SPRAY-DRYING AS A METHOD FOR MICROPARTICULATE MODIFIED RELEASE SYSTEMS PREPARATION", Drying 2004-Proceedings of the 14th International Drying Symposium, vol. B, 2004, pages 1112-1119,
- RYUICHI ANDO: "Cornstarch no Tokusei to Shin KakooRiyo Gijutsu", Agriculture & Livestock Industries Corp., 2010, XP055599618, Retrieved from the Internet: URL:https://www.alic.go.jp/starch/japan/ba sic/200805-01.html [retrieved on 2017-03-24]

## Description

### Technical Field

The present invention relates to a compacted pharmaceutical preparation in which an unpleasant taste of an acidic drug is masked, and more particularly relates to a compacted pharmaceutical preparation in which the effect of masking an unpleasant taste such as bitterness or mucosal irritancy of an acidic drug is high, and also the durability of the effect is excellent.

### Background Art

An acidic drug to be used in a pharmaceutical product generally has bitterness or astringency and also has strong mucosal irritancy and causes an unpleasant taste when it is ingested. There was a problem that the presence of such an unpleasant taste deteriorates the ingestion feeling of a patient. Therefore, as a method for masking such an unpleasant taste, various methods have been studied so far, and a method using a sweetener or the like, a method for performing coating or encapsulation, and the like are known. As the method using a sweetener, for example, a method in which sucralose which is a sweetener, a flavor, and a sugar alcohol are added to ibuprofen, followed by granulation is disclosed (PTL 1). Further, as a coating or encapsulation technique, for example, a method in which core particles containing ibuprofen are produced, and a multilayer coating including coating layers formed from a water-soluble polymer such as hydroxypropylmethyl cellulose and a water-insoluble polymer such as ethyl cellulose is applied to the core particles (PTL 2), and a method in which a core material containing ibuprofen is coated with a film material obtained by gelling a mixture of gelatin and an alginate, thereby forming microcapsules (PTL 3) are known.

In addition, a method in which by using unpleasant taste masking particles obtained by applying a first coating layer containing a slightly soluble polymer compound and a dissolution regulating agent to core particles, and also applying a second coating layer containing a slightly water-soluble polymer compound and a disintegrating agent thereon, breakage during tableting is suppressed, and an effect of suppressing bitterness in the mouth and an effect of fast dissolution in the stomach after tableting can be obtained simultaneously (PTL 4), and a method in which amorphous composite particles containing amorphous ibuprofen and aluminum hydroxide are prepared, followed by compacting the particles, whereby bitterness is suppressed (PTL 5) are proposed. In addition thereto, as a method for producing a compacted pharmaceutical preparation containing ibuprofen or the like, a method in which granulated drug particles obtained by stirring granulation while spraying or dropping an aqueous solution containing fructose on a powder containing a drug are prepared, followed by tableting the particles is disclosed (PTL 6). PTL 7 discloses ibuprofen tablet formulations with excellent mouthfeel and stability.

### Citation List

### Patent Literature

PTL 1: JP-A-2011-26310
PTL 2: JP-A-2010-37326
PTL 3: JP-A-2005-187416
PTL 4: JP-A-2001-106639
PTL 5: WO 2010/117035
PTL 6: JP-A-2011-37840
PTL 7: EP 1 491 184 A1

### Summary of Invention

### Technical Problem

However, when a compacted pharmaceutical preparation was prepared using masking particles by the above-mentioned techniques, a masking effect was not sufficiently obtained in some cases. In particular, in the case of a sublimable drug such as ibuprofen, even when an unpleasant taste was masked immediately after compaction, a tendency that the unpleasant taste appeared with the lapse of time was observed. In order to prevent deterioration of the masking performance due to such a compaction treatment as much as possible, it was also considered that the content of the masking particles in the compacted pharmaceutical preparation is reduced. However, there were problems that in order to ensure the dose, the design of the pharmaceutical preparation is accompanied by restrictions, for example, it is necessary to enlarge the shape or increase the number of pharmaceutical preparations to be ingested per dose, etc., and also ingestibility is lowered.

In view of this, an object of the present invention is to provide a compacted pharmaceutical preparation which has an excellent masking effect of reducing an unpleasant taste caused by an acidic drug and is capable of stably maintaining a good ingestion feeling without reducing the masking effect on the unpleasant taste over time.

### Solution to Problem

The present inventors made intensive studies for developing a method in which the bitterness or irritancy of a compacted pharmaceutical preparation containing an acidic drug is prevented, and the effect is not attenuated over time, and found that by combining an acidic drug with a gastric-soluble polymer, a water-insoluble polymer, and a water-soluble polymer to form core particles, further applying a film of an enteric-soluble polymer to the core particles to form coated particles, and formulating the coated particles into a pharmaceutical preparation by compaction, the bitterness or irritancy of the acidic drug is effectively suppressed, and also the effect is stably maintained for a long period of time, and thus, completed the present invention.

The present invention relates to the subject matter of claims 1 to 19. That is, the present invention is a compacted pharmaceutical preparation containing coated particles obtained by applying a film of an enteric-soluble polymer to core particles containing an acidic drug, a gastric-soluble polymer, a water-insoluble polymer, and a water-soluble polymer, wherein the acidic drug is ibuprofen, and the gastric-soluble polymer is polyvinyl acetal diethylamino acetate.

Further, the present invention is a method for producing a compacted pharmaceutical preparation, characterized in that core particles are obtained by granulating an acidic drug, a gastric-soluble polymer, a water-insoluble polymer, and a water-soluble polymer, and then, coated particles are obtained by applying a film of an enteric-soluble polymer to the core particles, and thereafter, the coated particles are compacted, wherein the acidic drug is ibuprofen, and the gastric-soluble polymer is polyvinyl acetal diethylamino acetate.

### Advantageous Effects of Invention

The compacted pharmaceutical preparation of the present invention can effectively suppress intense bitterness or mucosal irritancy that paralyzes the tongue caused by an acidic drug by combining the acidic drug with a gastric-soluble polymer, a water-insoluble polymer, and a water-soluble polymer to form core particles, and further applying a film of an enteric-soluble polymer to the core particles. Further, the effect is not reduced over time, and the masking effect on the unpleasant taste is stably maintained over a long period of time. In addition, there is no roughness or powdery texture when it is ingested and an ingestion feeling is also excellent, and moreover, the disintegrating property in the oral cavity is favorable.

### Description of Embodiments

The compacted pharmaceutical preparation of the present invention (hereinafter sometimes referred to as "pharmaceutical preparation of the present invention") contains coated particles (hereinafter sometimes referred to as "coated particles") obtained by applying a film of an enteric-soluble polymer to core particles (hereinafter sometimes referred to as "core particles") containing an acidic drug, a gastric-soluble polymer, a water-insoluble polymer, and a water-soluble polymer. The acidic drug is ibuprofen.

In the pharmaceutical preparation of present invention, the content of the acidic drug is not particularly limited and can be appropriately determined according to the tolerated dose in oral administration of each drug, or the like. For example, ibuprofen is contained in the core particles in an amount of preferably 1 to 98 mass% (hereinafter simply referred to as "%"), more preferably 50 to 70%. The content thereof in the coated particles is preferably from 1 to 95%, more preferably from 45 to 65%. Further, the content of the acidic drug in the coated particles with respect to the total mass of the compacted pharmaceutical preparation is preferably from 1 to 90%, more preferably from 25 to 55%.

The core particles constituting the pharmaceutical preparation of the present invention contains a gastric-soluble polymer, a water-insoluble polymer, and a water-soluble polymer other than the above-mentioned acidic drug.

The gastric-soluble polymer is, because the masking effect and the durability thereof and the like are excellent, polyvinyl acetal diethylamino acetate.

In the pharmaceutical preparation of the present invention, the content of the gastric-soluble polymer is not particularly limited, however, for example, the content of the gastric-soluble polymer in the core particles is preferably from 0.1 to 50% in terms of solid content from the viewpoint of the masking effect and the durability thereof, and the like, more preferably from 0.2 to 25%, further more preferably from 0.5 to 5%. The content of the gastric-soluble polymer in the coated particles is preferably from 0.1 to 45%, more preferably from 0.2 to 20%, further more preferably from 0.5 to 4%. The content of the gastric-soluble polymer in the coated particles with respect to the total mass of the compacted pharmaceutical preparation is preferably from 0.1 to 40%, more preferably from 0.2 to 15%, further more preferably from 0.4 to 3%.

Further, the water-insoluble polymer is not particularly limited, however, preferred examples thereof include water-insoluble methacrylic acid-based polymer compounds such as an ethyl acrylate-methyl methacrylate copolymer (also known as an ethyl acrylate-methyl methacrylate copolymer dispersion liquid, for example, Eudragit^{®} NE30D), and an ethyl acrylate-methyl methacrylate-ethyl methacrylate trimethylammonium chloride copolymer (also known as aminoalkyl methacrylate copolymer RS, for example, Eudragit ^{®} RS100, Eudragit^{®} RSPO, Eudragit^{®} RL, or Eudragit^{®} RLPO, and also known as an aminoalkyl methacrylate copolymer RS aqueous dispersion liquid, for example, Eudragit^{®} RS30D or Eudragit^{®} RL30D), and water-insoluble cellulose-based polymer compounds such as ethyl cellulose (for example, Ethocel^{®} or Aquacoat^{®} ), crystalline cellulose (for example, Ceolus, ^{®} Avicel, ^{®} Celphere, ^{®} or Farmacel ^{®} ), and low-substituted hydroxypropyl cellulose (for example, L-HPC), and of these, one type or two or more types can be used. In particular, it is preferred to use a water-insoluble methacrylic acid-based polymer compound and a water-insoluble cellulose-based polymer compound in combination because the masking effect and the durability thereof are excellent, and among these, it is preferred to use an ethyl acrylate-methyl methacrylate copolymer and crystalline cellulose in combination. The mass content ratio of the ethyl acrylate-methyl methacrylate copolymer to the crystalline cellulose in the core particles is preferably from 1:5 to 1:200 (ethyl acrylate-methyl methacrylate copolymer : crystalline cellulose) in terms of solid content, more preferably from 1:10 to 1:50, particularly preferably from 1:25 to 1:30. Also in the coated particles, it is preferred to set the mass content ratio in the same manner.

In the pharmaceutical preparation of the present invention, the content of the water-insoluble polymer is not particularly limited, however, for example, the content thereof in the core particles is preferably from 1 to 70% in terms of solid content, more preferably from 10 to 60%, further more preferably from 20 to 50%. The content thereof in the coated particles is preferably from 1 to 60%, more preferably from 10 to 50%, further more preferably from 20 to 40%. The content of the water-insoluble polymer in the coated particles with respect to the total mass of the compacted pharmaceutical preparation is preferably from 1 to 40%, more preferably from 5 to 35%, further more preferably from 10 to 30%.

The water-soluble polymer is not particularly limited, however, preferred examples thereof include methylcellulose, hypromellose, hydroxypropyl cellulose, polyethylene glycol, polyvinyl alcohol (a partially saponified product), and polyvinylpyrrolidone, and of these, one type or two or more types can be used. Among these, polyvinyl alcohol (a partially saponified product) is preferably used because the masking effect and the durability thereof are excellent.

In the pharmaceutical preparation of the present invention, the content of the water-soluble polymer is not particularly limited, however, for example, the content thereof in the core particles is preferably from 0.1 to 12% in terms of solid content, more preferably from 0.2 to 5%, further more preferably from 0.5 to 3%. The content thereof in the coated particles is preferably from 0.1 to 10%, more preferably from 0.2 to 4%, further more preferably from 0.5 to 2%. Further, the content of the water-soluble polymer in the coated particles with respect to the total mass of the compacted pharmaceutical preparation is preferably from 0.02 to 5%, more preferably from 0.05 to 2%, further more preferably from 0.1 to 1.3%.

In the pharmaceutical preparation of the present invention, to the core particles, further a pH adjusting agent can be added as needed. Specific examples thereof include substances described as a pH adjusting agent in Japanese Pharmaceutical Excipients Directory 2007 (edited by Japan Pharmaceutical Excipients Council, Yakuji Nippo, Ltd.) such as adipic acid, citric acid, sodium citrate, sodium dihydrogen citrate, glycine, succinic acid, tartaric acid, D-tartaric acid, L-tartaric acid, maleic acid, anhydrous citric acid, and DL-malic acid. By adding a pH adjusting agent, the masking property is enhanced, and also an effect of improving the manufacturability of the core particles is obtained.

In the pharmaceutical preparation of the present invention, the content of the pH adjusting agent is not particularly limited, however, for example, the content of the pH adjusting agent in the core particles is preferably from 0.1 to 12% in terms of solid content, more preferably from 0.2 to 5%, further more preferably from 0.5 to 3%. The content of the pH adjusting agent in the coated particles is preferably from 0.1 to 10%, more preferably from 0.2 to 4%, further more preferably from 0.5 to 2%. Further, the content of the pH adjusting agent in the coated particles with respect to the total mass of the compacted pharmaceutical preparation is preferably from 0.02 to 5%, more preferably from 0.05 to 2%, further more preferably from 0.1 to 1.3%.

The enteric-soluble polymer for coating the core particles is not particularly limited, however, examples thereof include enteric-soluble methacrylic acid-based polymer compounds such as a methacrylic acid-ethyl acrylate copolymer (also known as methacrylate copolymer LD, for example, Eudragit ^{®} L30D-55 or Eudragit ^{®} L100-55), a methacrylic acid-methyl methacrylate copolymer (also known as methacrylate copolymer L, for example, Eudragit ^{®} L100, and also known as methacrylate copolymer S, for example, Eudragit S100), and a methyl acrylate-methyl methacrylate-methacrylic acid copolymer (for example, Eudragit ^{®} FS30D) and enteric-soluble cellulose-based polymer compounds such as cellulose acetate phthalate (for example, CAP, cellulose acetate phthalate), hypromellose phthalate (for example, HP-55), hypromellose acetate succinate, polyvinyl acetate phthalate, and carboxymethylethyl cellulose, and of these, one type or two or more types can be used. Among these, an enteric-soluble methacrylic acid-ethyl acrylate copolymer is preferably used because the masking effect and the durability thereof, and the like are excellent.

In the pharmaceutical preparation of the present invention, the content of the enteric-soluble polymer is not particularly limited, however, from the viewpoint of the masking effect and the durability thereof, for example, the content of the enteric-soluble polymer in the coated particles is preferably from 1 to 30% in terms of solid content, more preferably from 2 to 20%, further more preferably from 5 to 16%. The content of the enteric-soluble polymer in the coated particles with respect to the total mass of the compacted pharmaceutical preparation is preferably from 0.5 to 25%, more preferably from 1 to 16%, further more preferably from 2 to 8%.

In the pharmaceutical preparation of the present invention, to the film of the enteric-soluble polymer, other than the enteric-soluble polymer, a coating agent which can be generally added to a coating film such as talc, magnesium stearate, calcium stearate, light anhydrous silicic acid, polyethylene glycol, triethyl citrate , stearic acid, hydrated silicon dioxide, or titanium oxide (Japanese Pharmaceutical Excipients Directory 2007 edited by Japan Pharmaceutical Excipients Council, Yakuji Nippo, Ltd.) or a pharmaceutical additive described as a plasticizer, an anti-adhesion agent, a lubricant, a taste masking agent, a defoaming agent, a flavoring agent, a flavor, a coloring agent, a pH adjusting agent, an excipient, a dispersant, a disintegrating agent, an aromatic agent, a preservative, a fluidizing agent, or the like can be added. Specific examples thereof include the same pharmaceutical additives to be added during compaction described below.

In the pharmaceutical preparation of the present invention, the coating amount of the film of the enteric-soluble polymer to be applied to the core particles is set to preferably 2 to 200 parts by mass, more preferably 4 to 100 parts by mass, particularly preferably 6 to 20 parts by mass with respect to 100 parts by mass of the core particles .

The pharmaceutical preparation of the present invention can be produced according to a conventional method. For example, first, the acidic drug, the gastric-soluble polymer, the water-insoluble polymer, and the water-soluble polymer, and according to need, the pH adjusting agent are added, followed by granulation, whereby core particles are prepared. On the core particles, a coating liquid containing the enteric-soluble polymer is sprayed or the like, whereby a film is formed. Further, the obtained coated particles are compacted along with other pharmaceutical additives, whereby the pharmaceutical preparation of the present invention is prepared. Examples of such pharmaceutical additives (hereinafter sometimes referred to as "tableting components") other than the coated particles to be added during compaction include an excipient, a fluidity improver, a lubricant, a binder, a disintegrating agent, a taste masking agent, a sweetener, and a flavor to be generally used in a tablet. These additives are described in PFSB/ELD Notification No. 1204-1 (Pharmaceutical Administration and Regulations), Japanese Pharmaceutical Excipients Directory 2007 (edited by Japan Pharmaceutical Excipients Council, Yakuji Nippo, Ltd.), and Eighth Edition Japan's Specifications and Standards for Food Additives (Japan Food Additives Association).

Examples of the excipient include lactose, mannitol, erythritol, starch, corn starch, pregelatinized starch, partially pregelatinized starch, crystalline cellulose, low-substituted hydroxypropyl cellulose, hydroxypropyl cellulose, refined white sugar, a sugar alcohol, light anhydrous silicic acid, calcium silicate, titanium oxide, and precipitated calcium carbonate. Among these excipients, one type or two or more types can be used.

Examples of the binder include gelatin, gum Arabic powder, methyl cellulose, carboxymethyl cellulose, sodium carboxymethyl cellulose, hydroxypropyl cellulose, hypromellose, polyvinylpyrrolidone, polyvinyl alcohol, a polyvinyl alcohol-acrylic acid-methyl methacrylate copolymer, a polyvinyl alcohol-polyethylene glycol graft copolymer, pullulan, dextrin, carboxymethyl cellulose calcium, sodium carboxymethyl cellulose, shellac, a carboxyvinyl polymer, sodium carboxymethyl starch, carboxymethylethyl cellulose, and cellulose acetate phthalate. Among these binders, one type or two or more types can be used.

Examples of the disintegrating agent include croscarmellose sodium, crospovidone, crosslinked insoluble polyvinylpyrrolidone, carmellose calcium, sodium carboxymethyl starch, potato starch, corn starch, and pregelatinized starch. Among these disintegrating agents, one type or two or more types can be used.

Examples of the lubricant include talc, stearic acid, magnesium stearate, calcium stearate, a sucrose fatty acid ester, and polyethylene glycol. Among these lubricants, one type or two or more types can be used.

Examples of the sweetener include aspartame, acesulfame potassium, xylitol, licorice, saccharin, and maltitol.

Examples of the taste masking agent include DL-malic acid, citric acid, and tartaric acid.

The content of the tableting component in the pharmaceutical preparation of the present invention is not particularly limited and can be appropriately determined according to the tolerated dose in oral administration of each drug, or the like. For example, the content of the tableting component in the compacted pharmaceutical preparation is preferably from 10 to 90%, more preferably from 25 to 60%, particularly preferably from 35 to 45%.

The pharmaceutical preparation of the present invention can be formulated into an orally disintegrating tablet, a chewable tablet, an uncoated tablet, or the like by arbitrarily controlling the tableting component. For example, in the case where the drug is ibuprofen, by using a tableting component composed of an excipient such as a cellulose, a starch, or a silicic anhydride in an amount of 75 to 98%, a lubricant such as talc or magnesium stearate in an amount of 1 to 5%, a sweetener such as acesulfame potassium or aspartame in an amount of 2 to 10%, a taste masking agent such as DL-malic acid in an amount of 2 to 8%, and a flavor in an amount of 0.1 to 2% as the tableting component, an orally disintegrating tablet, in which the disintegrating time is very short, an unpleasant taste such as bitterness or tingling of the drug is masked, and further, there is no roughness or powdery texture, and also the masking performance does not change over a long period of time, for example, a year and a half or longer can be formed.

The pharmaceutical preparation of the present invention can be produced by granulating an acidic drug, a gastric-soluble polymer, a water-insoluble polymer, and a water-soluble polymer, thereby obtaining core particles, and then applying a film of an enteric-soluble polymer to the core particles, thereby obtaining coated particles, and thereafter compacting the coated particles. More specifically, first, an acidic drug, a gastric-soluble polymer, a water-insoluble polymer, a water-soluble polymer, and according to need, a pH adjusting agent are mixed, and thereafter, to this mixed powder, a solvent such as water or a lower alcohol, or a mixed aqueous solution of these is added, followed by kneading and granulation. At this time, the gastric-soluble polymer, the water-insoluble polymer, the water-soluble polymer, and the pH adjusting agent are partially or entirely dissolved or dispersed in water or a lower alcohol, a mixed aqueous solution of these, or the like, and the resulting solution or dispersion may be added. The granulation can be performed using a method of performing granulation according to a known granulation method such as a high-speed stirring granulation method or a fluidized bed granulation method, a method in which mixing, kneading, pressing, heating, cooling, extrusion, and granulation are performed using an extruder such as a twin-screw extruder, or the like. Incidentally, as the lower alcohol, a primary alcohol having 2 to 3 carbon atoms such as ethanol or isopropanol can be used. The gastric-soluble polymer, the water-insoluble polymer, and the water-soluble polymer may be granulated after these polymers are put together, and dissolved or dispersed in the solvent, and then added to the mixed powder, or may be granulated after each polymer is independently dissolved or dispersed in the solvent, and each solution or dispersion is added to the mixed powder. The granulated material formed by this granulation step is subsequently dried by a fluidized-bed dryer or the like, whereby core particles are prepared. At this time, according to need, grinding, sieving, sizing, or the like may be performed before or after drying. As for the particle diameters of the core particles, the average particle diameter is from about 20 to 1000 µm, preferably from 50 to 700 µm. Incidentally, the average particle diameter of the core particles is a value measured from the mass/volume distribution.

Subsequently, the obtained core particles are fluidized in a fluidized bed, and a coating liquid obtained by dissolving or dispersing the enteric-soluble polymer in an appropriate solvent such as water or a lower alcohol, or a mixed aqueous solution of these is sprayed in the fluidized bed, whereby coated particles coated with a film of the enteric-soluble polymer are prepared. To the coating liquid, a pharmaceutical additive such as a plasticizer may be added according to need as described above.

The coated particles are mixed with a filler powder component composed of a pharmaceutical additive to be generally used in a tablet such as an excipient, a disintegrating agent, a lubricant, a taste masking agent, a sweetener, or a flavor, thereby preparing a tableting powder, and the tableting powder is compacted using a single punch tableting machine, a rotary tableting machine, or the like, whereby the compacted pharmaceutical preparation of the present invention is produced. The pressure during compaction is preferably from 1 to 110 kN, more preferably from 2 to 90 kN. Further, the tablet after compaction is preferably shaped into a form which is easily ingested such as a round tablet with a diameter of approximately about 7 to 15 mm.

The thus produced compacted pharmaceutical preparation of the present invention does not require a complicated step such as multilayer coating or encapsulation, or a special apparatus, and therefore, the production cost is also low, and the processing is easy. Further, the gastric-soluble polymer, the water-insoluble polymer, and the water-soluble polymer are added to the acidic drug, thereby forming core particles, and the core particles are coated with the film of the enteric-soluble polymer, thereby forming coated particles, followed by compaction, and therefore, the bitterness or astringency of the acidic drug is suppressed, no roughness or powdery texture is felt so that the ingestion feeling is excellent, and further, the effect of suppressing the bitterness or astringency is maintained for a long period of time, and thus, the stability is also excellent. In addition, the disintegrating property in the oral cavity is also excellent, and thus, the preparation is favorable as an orally disintegrating tablet or the like.

As particularly preferred embodiments of the pharmaceutical preparation of the present invention described above, compositions shown in the following Table 1 can be exemplified.

**[Table 1]**

| | Content in core particles | Content in coated particles | Content in coated particles with respect to total mass of compacted pharmaceutical preparation |
|---|---|---|---|
| Acidic drug | 50 to 70 mass% | 45 to 65 mass% | 25 to 55 mass% |
| Gastric-soluble polymer | 0.5 to 5 mass% | 0.5 to 4 mass% | 0.4 to 3 mass% |
| Water-insoluble polymer | 20 to 50 mass% | 20 to 40 mass% | 10 to 30 mass% |
| Water-soluble polymer | 0.5 to 3 mass% | 0.5 to 2 mass% | 0.1 to 1.3 mass% |
| pH adjusting agent | 0.5 to 3 mass% | 0.5 to 2 mass% | 0.1 to 1.3 mass% |
| Enteric-soluble polymer | - | 5 to 16 mass% | 2 to 8 mass% |
| | | | 35 to 45 mass% |
| Tableting component | - | - | (Content in compacted pharmaceutical preparation) |

### Examples

Next, the present invention will be more specifically described by showing Examples and Comparative Examples, however, the present invention is by no means limited thereto.

### Example 1

Granulation was performed by placing 600 g of ibuprofen, 330 g of crystalline cellulose, 24 g of polyvinyl acetal diethylamino acetate, 40 g of an ethyl acrylate-methyl methacrylate copolymer dispersion liquid (solid content: 30%) , 12 g of DL-malic acid, 12 g of polyvinyl alcohol (a partially saponified product) , and 100 g of purified water in a high-speed stirring-type mixing granulator. Subsequently, the obtained granulated material was dried using a fluidized-bed dryer, and sized using a sizing machine, whereby core particles having an average particle diameter of 235 µm were obtained. 950 g of the core particles were taken out, and a coating liquid obtained by uniformly mixing 450 g of a methacrylate copolymer LD dispersion liquid (solid content: 30%), 13.5 g of triethyl citrate, 13.5 g of light anhydrous silicic acid, and 333 g of purified water was sprayed thereon in the fluidized bed until the increase in mass of the core particles reached 10.9%, whereby coated particles were obtained. 450 g of the coated particles was uniformly mixed with 139.4 g of crystalline cellulose, 110.7 g of corn starch, 7.4 g of acesulfame potassium, 7.4 g of aspartame, 11.1 g of DL-malic acid, 0.7 g of a flavor, 3.7 g of light anhydrous silicic acid, 3.7 g of talc, and 3.7 g of magnesium stearate, whereby a tableting powder was prepared. This tableting powder was compacted by a rotary tableting machine using a mortar and a pestle with a diameter of 12 mm, whereby a tablet having a thickness of about 4.6 mm and a mass per tablet of 600 mg, and containing 200 mg of ibuprofen was obtained.

The contents (mass%) of the respective components in this tablet are shown in the following Table 2.

**[Table 2]**

| | Content in core particles | Content in coated particles | Content in coated particles with respect to total mass of compacted pharmaceutical preparation |
|---|---|---|---|
| Acidic drug | 60.6 | 54.7 | 33.3 |
| Gastric-soluble polymer | 2.4 | 2.2 | 1.3 |
| Water-insoluble polymer | 34.6 | 31.2 | 19.0 |
| Water-soluble polymer | 1.2 | 1.1 | 0.7 |
| pH adjusting agent | 1.2 | 1.1 | 0.7 |
| Enteric-soluble polymer | - | 8.2 | 5.0 |
| | | | 39.0 |
| Tableting component | - | - | (Content in compacted pharmaceutical preparation) |

### Test Example 1

With respect to the tablet of Example 1 after production, a sensory test for an unpleasant taste was performed by 20 test subjects. Each test subject ingested the tablet by placing it on the tongue without chewing and leaving it to stand until disintegration occurred, and evaluated bitterness or tingling, and a roughness or a powdery texture when it was ingested according to the following criteria, respectively. Further, each test subject also measured the time until the tablet disintegrated in the mouth after ingestion. The evaluation results of the respective test subjects and the averages are shown in Table 3.

### <Evaluation Criteria for Masking of Bitterness and Tingling> (Score) (Contents)

4 points: The tablet has a very high masking effect.
3 points: The tablet has a masking effect.
2 points: The tablet has a slight masking effect.
1 point: The tablet has a very slight masking effect.
0 points: The tablet has no masking effect.

### <Evaluation Criteria for Ingestion Feeling as to Roughness or Powdery Texture>

### (Score) (Contents)

4 points: The tablet has no roughness or powdery texture.
3 points: The tablet has a very slight roughness or powdery texture.
2 points: The tablet has a slight roughness or powdery texture.
1 point: The tablet has a roughness or powdery texture.
0 points: The tablet has a very strong roughness or powdery texture.

**[Table 3]**

| Test subject No. | Masking evaluation | Ingestion feeling evaluation | Oral disintegrating time (sec) |
|---|---|---|---|
| 1 | 4 | 4 | 14 |
| 2 | 4 | 3 | 12 |
| 3 | 4 | 3 | 10 |
| 4 | 4 | 3 | 13 |
| 5 | 4 | 3 | 9 |
| 6 | 3 | 3 | 8 |
| 7 | 3 | 3 | 20 |
| 8 | 4 | 4 | 10 |
| 9 | 4 | 4 | 20 |
| 10 | 4 | 3 | 18 |
| 11 | 4 | 3 | 28 |
| 12 | 4 | 3 | 10 |
| 13 | 3 | 4 | 5 |
| 14 | 4 | 2 | 12 |
| 15 | 4 | 2 | 10 |
| 16 | 4 | 3 | 11 |
| 17 | 4 | 3 | 9 |
| 18 | 4 | 3 | 40 |
| 19 | 3 | 3 | 5 |
| 20 | 4 | 4 | 13 |
| Average | 3.8 | 3.15 | 13.85 |

As shown in Table 3, in the tablet of Example 1, the unpleasant taste such as bitterness or strong irritancy of ibuprofen was reduced, and the masking effect was excellent. Further, almost no roughness or powdery texture was felt and the ingestion feeling was also favorable. Further, the tablet disintegrated in a short time in the oral cavity and the disintegrating property was also excellent.

### Example 2

A tableting powder was produced in the same manner as in Example 1, and the tableting powder was compacted by a rotary tableting machine using a mortar and a pestle with a diameter of 9 mm, whereby a tablet having a thickness of about 4 mm and a mass per tablet of 300 mg, and containing 100 mg of ibuprofen was obtained.

### Example 3

A tableting powder was produced in the same manner as in Example 1, and the tableting powder was compacted by a rotary tableting machine using a mortar and a pestle with a diameter of 11 mm, whereby a tablet having a thickness of about 4.1 mm and a mass per tablet of 450 mg, and containing 150 mg of ibuprofen was obtained.

### Example 4

A tableting powder was produced in the same manner as in Example 1, and the tableting powder was compacted by a rotary tableting machine using a mortar and a pestle with a diameter of 15 mm, whereby a tablet having a thickness of about 5.8 mm and a mass per tablet of 1200 mg, and containing 400 mg of ibuprofen was obtained.

### Test Example 2

The tablet obtained in Example 1 was packaged in PTP and placed in an aluminum inner bag together with a desiccant, and the bag was hermetically sealed. After the tablet was stored for 24 months at room temperature (a thermostatic chamber at 25°C and 40% RH) . Thereafter, a sensory test was performed by 12 test subjects according to the same evaluation criteria as in Test Example 1. The evaluation results of the respective test subjects and the averages are shown in Table 4.

**[Table 4]**

| Test subject No. | Masking evaluation | Ingestion feeling evaluation | Oral disintegrating time (sec) |
|---|---|---|---|
| 1 | 4 | 4 | 15 |
| 2 | 4 | 4 | 11 |
| 3 | 4 | 3 | 11 |
| 4 | 4 | 4 | 10 |
| 5 | 4 | 4 | 9 |
| 6 | 3 | 3 | 7 |
| 7 | 4 | 3 | 18 |
| 8 | 4 | 4 | 8 |
| 9 | 3 | 4 | 22 |
| 10 | 4 | 3 | 12 |
| 11 | 3 | 3 | 26 |
| 12 | 4 | 3 | 8 |
| Average | 3.75 | 3.50 | 13.1 |

As shown in Table 4, it was revealed that in the tablet of Example 1, the masking effect on the unpleasant taste of ibuprofen is maintained with almost no change from immediately after production even after it was stored at room temperature for 24 months . Further, almost no roughness or powdery texture was felt and a good ingestion feeling was also maintained. Further, the disintegrating time was also very short, and no change in the disintegrating property was observed in comparison with the initial state.

### Industrial Applicability

The compacted pharmaceutical preparation of the present invention has an excellent effect of masking the bitterness, irritation, or the like of an acidic drug and also has excellent durability of the effect, and further has a good ingestion feeling, and therefore can be favorably utilized as an orally disintegrating tablet or the like containing an acidic drug having an unpleasant taste as an active ingredient.

## Claims

1. A compacted pharmaceutical preparation, comprising coated particles obtained by applying a film of an enteric-soluble polymer to core particles containing an acidic drug, a gastric-soluble polymer, a water-insoluble polymer, and a water-soluble polymer,
wherein the acidic drug is ibuprofen, and
wherein the gastric-soluble polymer is polyvinyl acetal diethylamino acetate.

2. The compacted pharmaceutical preparation according to claim 1, wherein the water-insoluble polymer is one type or two or more types selected from the group consisting of water-insoluble methacrylic acid-based polymer compounds and water-insoluble cellulose-based polymer compounds.

3. The compacted pharmaceutical preparation according to claim 2, wherein the water-insoluble polymer contains an ethyl acrylate-methyl methacrylate copolymer and crystalline cellulose.

4. The compacted pharmaceutical preparation according to claim 3, wherein the mass content ratio of the ethyl acrylate-methyl methacrylate copolymer to the crystalline cellulose in the core particles is from 1:5 to 1:200.

5. The compacted pharmaceutical preparation according to any one of claims 1 to 4, wherein the water-soluble polymer is one type or two or more types selected from the group consisting of methylcellulose, hypromellose, hydroxypropyl cellulose, polyethylene glycol, polyvinyl alcohol (a partially saponified product), and polyvinylpyrrolidone.

6. The compacted pharmaceutical preparation according to any one of claims 1 to 5, wherein a pH adjusting agent is further contained in the core particles.

7. The compacted pharmaceutical preparation according to any one of claims 1 to 6, wherein in the core particles, the content of the gastric-soluble polymer is from 0.1 to 50 mass%, the content of the water-insoluble polymer is from 1 to 70 mass%, and the content of the water-soluble polymer is from 0.1 to 12 mass%.

8. The compacted pharmaceutical preparation according to any one of claims 1 to 7, wherein the enteric-soluble polymer is one type or two or more types selected from the group consisting of enteric-soluble methacrylic acid-based polymer compounds and enteric-soluble cellulose-based polymer compounds.

9. The compacted pharmaceutical preparation according to any one of claims 1 to 8, wherein the content of the enteric-soluble polymer in the coated particles is from 1 to 30 mass%.

10. A method for producing a compacted pharmaceutical preparation, **characterized in that** core particles are obtained by granulating an acidic drug, a gastric-soluble polymer, a water-insoluble polymer, and a water-soluble polymer, and then, coated particles are obtained by applying a film of an enteric-soluble polymer to the core particles, and thereafter, the coated particles are compacted,
wherein the acidic drug is ibuprofen, and
wherein the gastric-soluble polymer is polyvinyl acetal diethylamino acetate.

11. The method for producing a compacted pharmaceutical preparation according to claim 10, wherein the water-insoluble polymer is one type or two or more types selected from the group consisting of water-insoluble methacrylic acid-based polymer compounds and water-insoluble cellulose-based polymer compounds.

12. The method for producing a compacted pharmaceutical preparation according to claim 10 or 11, wherein the water-soluble polymer is one type or two or more types selected from the group consisting of methylcellulose, hypromellose, hydroxypropyl cellulose, polyethylene glycol, polyvinyl alcohol (a partially saponified product), and polyvinylpyrrolidone.

13. The method for producing a compacted pharmaceutical preparation according to any one of claims 10 to 12, wherein the enteric-soluble polymer is one type or two or more types selected from the group consisting of enteric-soluble methacrylic acid-based polymer compounds and enteric-soluble cellulose-based polymer compounds.

14. The method for producing a compacted pharmaceutical preparation according to any one of claims 10 to 13, wherein the film of an enteric-soluble polymer is applied so that the content of the enteric-soluble polymer in the coated particles is from 1 to 30 mass%.

15. A method for masking an unpleasant taste of a compacted pharmaceutical preparation, **characterized in that** core particles are obtained by granulating an acidic drug, a gastric-soluble polymer, a water-insoluble polymer, and a water-soluble polymer, and then, coated particles are obtained by applying a film of an enteric-soluble polymer to the core particles, and thereafter, the coated particles are compacted,
wherein the acidic drug is ibuprofen, and
wherein the gastric-soluble polymer is polyvinyl acetal diethylamino acetate.

16. The method for masking an unpleasant taste of a compacted pharmaceutical preparation according to claim 15, wherein the water-insoluble polymer is one type or two or more types selected from the group consisting of water-insoluble methacrylic acid-based polymer compounds and water-insoluble cellulose-based polymer compounds.

17. The method for masking an unpleasant taste of a compacted pharmaceutical preparation according claim 15 or 16, wherein the water-soluble polymer is one type or two or more types selected from the group consisting of methylcellulose, hypromellose, hydroxypropyl cellulose, polyethylene glycol, polyvinyl alcohol (a partially saponified product), and polyvinylpyrrolidone.

18. The method for masking an unpleasant taste of a compacted pharmaceutical preparation according to any one of claims 15 to 17, wherein the enteric-soluble polymer is one type or two or more types selected from the group consisting of enteric-soluble methacrylic acid-based polymer compounds and enteric-soluble cellulose-based polymer compounds.

19. The method for masking an unpleasant taste of a compacted pharmaceutical preparation according to any one of claims 15 to 18, wherein the film of an enteric-soluble polymer is applied so that the content of the enteric-soluble polymer in the coated particles is from 1 to 30 mass%.

## Patentansprüche

1. Kompaktiertes pharmazeutisches Präparat, umfassend beschichtete Partikel, die durch Aufbringen eines Films aus einem darmlöslichen Polymer auf Kernpartikel erhalten werden, die ein saures Arzneimittel, ein magenlösliches Polymer, ein wasserunlösliches Polymer und ein wasserlösliches Polymer enthalten, wobei das saure Arzneimittel Ibuprofen ist und wobei das magenlösliche Polymer Polyvinylacetal-Diethylaminoacetat ist.

2. Kompaktiertes pharmazeutisches Präparat nach Anspruch 1, wobei das wasserunlösliche Polymer ein Typ ist oder zwei oder mehr Typen sind, ausgewählt aus der Gruppe, bestehend aus wasserunlöslichen Polymerverbindungen auf Basis von Methacrylsäure und wasserunlöslichen Polymerverbindungen auf Basis von Cellulose.

3. Kompaktiertes pharmazeutisches Präparat nach Anspruch 2, wobei das wasserunlösliche Polymer ein Ethylacrylat-Methylmethacrylat-Copolymer und kristalline Cellulose enthält.

4. Kompaktiertes pharmazeutisches Präparat nach Anspruch 3, wobei das Verhältnis des Massengehalts des Ethylacrylat-Methylmethacrylat-Copolymers zu der kristallinen Cellulose in den Kernpartikeln von 1:5 bis 1:200 beträgt.

5. Kompaktiertes pharmazeutisches Präparat nach einem der Ansprüche 1 bis 4, wobei das wasserlösliche Polymer ein Typ ist oder zwei oder mehr Typen sind, ausgewählt aus der Gruppe, bestehend aus Methylcellulose, Hypromellose, Hydroxypropylcellulose, Polyethylenglycol, Polyvinylalkohol (ein teilweise verseiftes Produkt) und Polyvinylpyrrolidon.

6. Kompaktiertes pharmazeutisches Präparat nach einem der Ansprüche 1 bis 5, wobei in den Kernpartikeln ferner ein Mittel zur Einstellung des pH-Werts enthalten ist.

7. Kompaktiertes pharmazeutisches Präparat nach einem der Ansprüche 1 bis 6, wobei der Gehalt an magenlöslichem Polymer in den Kernpartikeln von 0,1 bis 50 Massenprozent, der Gehalt an wasserunlöslichem Polymer von 1 bis 70 Massenprozent und der Gehalt an wasserlöslichem Polymer von 0,1 bis 12 Massenprozent beträgt.

8. Kompaktiertes pharmazeutisches Präparat nach einem der Ansprüche 1 bis 7, wobei das darmlösliche Polymer ein Typ ist oder zwei oder mehr Typen sind, ausgewählt aus der Gruppe, bestehend aus darmlöslichen Polymerverbindungen auf Basis von Methacrylsäure und darmlöslichen Polymerverbindungen auf Basis von Cellulose.

9. Kompaktiertes pharmazeutisches Präparat nach einem der Ansprüche 1 bis 8, wobei der Gehalt an darmlöslichem Polymer in den beschichteten Partikeln von 1 bis 30 Massenprozent beträgt.

10. Verfahren zur Herstellung eines kompaktierten pharmazeutischen Präparats, **dadurch gekennzeichnet, dass** die Kernpartikel durch Granulieren eines sauren Arzneimittels, eines magenlöslichen Polymers, eines wasserunlöslichen Polymers und eines wasserlöslichen Polymers erhalten werden und dann beschichtete Partikel erhalten werden, indem ein Film aus einem darmlöslichen Polymer auf die Kernpartikel aufgebracht wird und die beschichteten Partikel nachfolgend kompaktiert werden, wobei das saure Arzneimittel Ibuprofen ist und wobei das magenlösliche Polymer Polyvinylacetal-Diethylaminoacetat ist.

11. Verfahren zur Herstellung eines kompaktierten pharmazeutischen Präparats nach Anspruch 10, wobei das wasserunlösliche Polymer ein Typ ist oder zwei oder mehr Typen sind, ausgewählt aus der Gruppe, bestehend aus wasserunlöslichen Polymerverbindungen auf Basis von Methacrylsäure und wasserunlöslichen Polymerverbindungen auf Basis von Cellulose.

12. Verfahren zur Herstellung eines kompaktierten pharmazeutischen Präparats nach Anspruch 10 oder 11, wobei das wasserlösliche Polymer ein Typ ist oder zwei oder mehr Typen sind, ausgewählt aus der Gruppe, bestehend aus Methylcellulose, Hypromellose, Hydroxypropylcellulose, Polyethylenglycol, Polyvinylalkohol (ein teilweise verseiftes Produkt) und Polyvinylpyrrolidon.

13. Verfahren zur Herstellung eines kompaktierten pharmazeutischen Präparats nach einem der Ansprüche 10 bis 12, wobei das darmlösliche Polymer ein Typ ist oder zwei oder mehr Typen sind, ausgewählt aus der Gruppe, bestehend aus darmlöslichen Polymerverbindungen auf Basis von Methacrylsäure und darmlöslichen Polymerverbindungen auf Basis von Cellulose.

14. Verfahren zur Herstellung eines kompaktierten pharmazeutischen Präparats nach einem der Ansprüche 10 bis 13, wobei der Film des darmlöslichen Polymers so aufgebracht wird, dass der Gehalt an darmlöslichem Polymer in den beschichteten Partikeln von 1 bis 30 Massenprozent beträgt.

15. Verfahren zum Maskieren eines unangenehmen Geschmacks eines kompaktierten pharmazeutischen Präparats, **dadurch gekennzeichnet, dass** die Kernpartikel durch Granulieren eines sauren Arzneimittels, eines magenlöslichen Polymers, eines wasserunlöslichen Polymers und eines wasserlöslichen Polymers erhalten werden und dann beschichtete Partikel erhalten werden, indem ein Film aus einem darmlöslichen Polymer auf die Kernpartikel aufgebracht wird und die beschichteten Partikel nachfolgend kompaktiert werden, wobei das saure Arzneimittel Ibuprofen ist und wobei das magenlösliche Polymer Polyvinylacetal-Diethylaminoacetat ist.

16. Verfahren zum Maskieren eines unangenehmen Geschmacks eines kompaktierten pharmazeutischen Präparats nach Anspruch 15, wobei das wasserunlösliche Polymer ein Typ ist oder zwei oder mehr Typen sind, ausgewählt aus der Gruppe, bestehend aus wasserunlöslichen Polymerverbindungen auf Basis von Methacrylsäure und wasserunlöslichen Polymerverbindungen auf Basis von Cellulose.

17. Verfahren zum Maskieren eines unangenehmen Geschmacks eines kompaktierten pharmazeutischen Präparats nach Anspruch 15 oder 16, wobei das wasserlösliche Polymer ein Typ ist oder zwei oder mehr Typen sind, ausgewählt aus der Gruppe, bestehend aus Methylcellulose, Hypromellose, Hydroxypropylcellulose, Polyethylenglycol, Polyvinylalkohol (ein teilweise verseiftes Produkt) und Polyvinylpyrrolidon.

18. Verfahren zum Maskieren eines unangenehmen Geschmacks eines kompaktierten pharmazeutischen Präparats nach einem der Ansprüche 15 bis 17, wobei das darmlösliche Polymer ein Typ ist oder zwei oder mehr Typen sind, ausgewählt aus der Gruppe, bestehend aus darmlöslichen Polymerverbindungen auf Basis von Methacrylsäure und darmlöslichen Polymerverbindungen auf Basis von Cellulose.

19. Verfahren zum Maskieren eines unangenehmen Geschmacks eines kompaktierten pharmazeutischen Präparats nach einem der Ansprüche 15 bis 18, wobei der Film des darmlöslichen Polymers so aufgebracht wird, dass der Gehalt an darmlöslichem Polymer in den beschichteten Partikeln von 1 bis 30 Massenprozent beträgt.

## Revendications

1. Préparation pharmaceutique compactée, comprenant des particules enrobées obtenues par l'application d'un film d'un polymère entérosoluble sur des particules de noyau contenant un médicament acide, un polymère gastrosoluble, un polymère insoluble dans l'eau et un polymère hydrosoluble,
dans laquelle le médicament acide est l'ibuprofène, et
dans laquelle le polymère gastrosoluble est un polyacétaldiéthylaminoacétate de vinyle.

2. Préparation pharmaceutique compactée selon la revendication 1, dans laquelle le polymère insoluble dans l'eau est un type ou deux types ou plus sélectionnés dans le groupe consistant en les composés polymères à base d'acide méthacrylique insolubles dans l'eau et les composés polymères à base de cellulose insolubles dans l'eau.

3. Préparation pharmaceutique compactée selon la revendication 2, dans laquelle le polymère insoluble dans l'eau contient un copolymère d'acrylate d'éthyle-méthacrylate de méthyle et une cellulose cristalline.

4. Préparation pharmaceutique compactée selon la revendication 3, dans laquelle le rapport de teneur massique du copolymère d'acrylate d'éthyle-méthacrylate de méthyle par rapport à la cellulose cristalline dans les particules de noyau est de 1:5 à 1:200.

5. Préparation pharmaceutique compactée selon l'une quelconque des revendications 1 à 4, dans laquelle le polymère hydrosoluble est un type ou deux types ou plus sélectionnés dans le groupe consistant en une méthylcellulose, une hypromellose, une hydroxypropyl-cellulose, un polyéthylèneglycol, un polyalcool vinylique (un produit partiellement saponifié) et une polyvinylpyrrolidone.

6. Préparation pharmaceutique compactée selon l'une quelconque des revendications 1 à 5, dans laquelle un agent d'ajustement de pH est en outre contenu dans les particules de noyau.

7. Préparation pharmaceutique compactée selon l'une quelconque des revendications 1 à 6, dans laquelle, dans les particules de noyau, la teneur en polymère gastrosoluble est de 0,1 à 50 % en masse, la teneur en polymère insoluble dans l'eau est de 1 à 70 % en masse, et la teneur en polymère hydrosoluble est de 0,1 à 12 % en masse.

8. Préparation pharmaceutique compactée selon l'une quelconque des revendications 1 à 7, dans laquelle le polymère entérosoluble est un type ou deux types ou plus sélectionnés dans le groupe consistant en les composés polymères entérosolubles à base d'acide méthacrylique et les composés polymères entérosolubles à base de cellulose.

9. Préparation pharmaceutique compactée selon l'une quelconque des revendications 1 à 8, dans laquelle la teneur en polymère entérosoluble dans les particules enrobées est de 1 à 30 % en masse.

10. Procédé de production d'une préparation pharmaceutique compactée, **caractérisé en ce que** des particules de noyau sont obtenues par la granulation d'un médicament acide, d'un polymère gastrosoluble, d'un polymère insoluble dans l'eau et d'un polymère hydrosoluble, puis des particules enrobées sont obtenues par l'application d'un film d'un polymère entérosoluble sur les particules de noyau, puis les particules enrobées sont compactées,
dans lequel le médicament acide est l'ibuprofène, et
dans lequel le polymère gastrosoluble est un polyacétaldiéthylaminoacétate de vinyle.

11. Procédé de production d'une préparation pharmaceutique compactée selon la revendication 10, dans lequel le polymère insoluble dans l'eau est un type ou deux types ou plus sélectionnés dans le groupe consistant en les composés polymères à base d'acide méthacrylique insolubles dans l'eau et les composés polymères à base de cellulose insolubles dans l'eau.

12. Procédé de production d'une préparation pharmaceutique compactée selon la revendication 10 ou 11, dans lequel le polymère hydrosoluble est un type ou deux types ou plus sélectionnés dans le groupe consistant en une méthylcellulose, une hypromellose, une hydroxypropylcellulose, un polyéthylèneglycol, un polyalcool vinylique (un produit partiellement saponifié) et une polyvinylpyrrolidone.

13. Procédé de production d'une préparation pharmaceutique compactée selon l'une quelconque des revendications 10 à 12, dans lequel le polymère entérosoluble est un type ou deux types ou plus sélectionnés dans le groupe consistant en les composés polymères entérosolubles à base d'acide méthacrylique et les composés polymères entérosolubles à base de cellulose.

14. Procédé de production d'une préparation pharmaceutique compactée selon l'une quelconque des revendications 10 à 13, dans lequel le film d'un polymère entérosoluble est appliqué de telle sorte que la teneur en polymère entérosoluble dans les particules enrobées est de 1 à 30 % en masse.

15. Procédé pour masquer un goût désagréable d'une préparation pharmaceutique compactée, **caractérisé en ce que** des particules de noyau sont obtenues par la granulation d'un médicament acide, d'un polymère gastrosoluble, d'un polymère insoluble dans l'eau et d'un polymère hydrosoluble, puis des particules enrobées sont obtenues par l'application d'un film d'un polymère entérosoluble sur les particules de noyau, puis les particules enrobées sont compactées,
dans lequel le médicament acide est l'ibuprofène, et
dans lequel le polymère gastrosoluble est un polyacétaldiéthylaminoacétate de vinyle.

16. Procédé pour masquer un goût désagréable d'une préparation pharmaceutique compactée selon la revendication 15, dans lequel le polymère insoluble dans l'eau est un type ou deux types ou plus sélectionnés dans le groupe consistant en les composés polymères à base d'acide méthacrylique insolubles dans l'eau et les composés polymères à base de cellulose insolubles dans l'eau.

17. Procédé pour masquer un goût désagréable d'une préparation pharmaceutique compactée selon la revendication 15 ou 16, dans lequel le polymère hydrosoluble est un type ou deux types ou plus sélectionnés dans le groupe consistant en une méthylcellulose, une hypromellose, une hydroxypropyl-cellulose, un polyéthylèneglycol, un polyalcool vinylique (un produit partiellement saponifié) et une polyvinylpyrrolidone.

18. Procédé pour masquer un goût désagréable d'une préparation pharmaceutique compactée selon l'une quelconque des revendications 15 à 17, dans lequel le polymère entérosoluble est un type ou deux types ou plus sélectionnés dans le groupe consistant en les composés polymères entérosolubles à base d'acide méthacrylique et les composés polymères entérosolubles à base de cellulose.

19. Procédé pour masquer un goût désagréable d'une préparation pharmaceutique compactée selon l'une quelconque des revendications 15 à 18, dans lequel le film d'un polymère entérosoluble est appliqué de telle sorte que la teneur en polymère entérosoluble dans les particules enrobées est de 1 à 30 % en masse.
